# EUROPÄISCHE PATENTSCHRIFT

(11) **EP 0 621 264 B1**
(45) Veröffentlichungstag und Bekanntmachung des Hinweises auf die Patenterteilung: **20.08.1997**
(21) Anmeldenummer: 94105661.6
(22) Anmeldetag: 13.04.1994
(51) Int. Cl.: C07C 251/48, C07C 69/736, C07C 45/46

(54) **Verfahren zur Herstellung von Aryl-benzylethern**
Process for the preparation of aryl-benzylethers
Procédé de préparation de aryl-benzyléthers

(30) Priorität: 19.04.1993 DE 4312637
(43) Veröffentlichungstag der Anmeldung: 26.10.1994
(73) Patentinhaber: BASF Aktiengesellschaft, 67063 Ludwigshafen (DE)
(72) Erfinder: Grammenos, Wassilios, Dr., D-67063 Ludwigshafen (DE); Siegel, Wolfgang, Dr., D-68167 Mannheim (DE); Oberdorf, Klaus, Dr., D-69117 Heidelberg (DE); Mueller, Bernd, Dr., D-67227 Frankenthal (DE); Sauter, Hubert, Dr., D-68167 Mannheim (DE); Doetzer, Reinhard, Dr., D-69469 Weinheim (DE)

(56) Entgegenhaltungen:
- EP-A- 0 334 096
- EUGEN MÜLLER 'Methoden der Organischen Chemie (Houben-Weyl) Band VII/2a' 1973 , GEORG THIEME VERLAG , STUTTGART * Seite 15, letzter Absatz - Seite 16, Zeile 1-5 *
- E. MÜLLER, Methoden der Organischen Chemie, Houben-Weyl, Band VII/2a, 1973, Georg Thieme Verlag, Stuttgart, DE, Seite 15, 16

## Beschreibung

Verfahren zur Herstellung von Aryl-benzylethern der allgemeinen Formel I 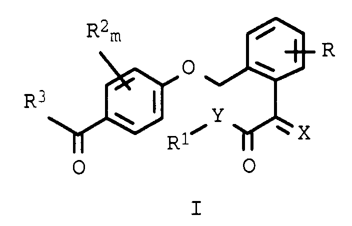 wobei die Variablen die folgende Bedeutung haben:
R
   Wasserstoff, Halogen, Cyano;
   C₁-C₄-Alkyl, C₁-C₄-Alkoxy;
X
   CH₂, CH-CH₃, CH-CH₂-CH₃, CH-OCH₃, N-OCH₃;
Y
   Sauerstoff, Schwefel, direkte Bindung oder Stickstoff, wobei der Stickstoff ein Wasserstoffatom, eine C₁-C₄-Alkylgruppe, eine C₁-C₄-Alkoxygruppe tragen kann;
m
   0, 1, 2 oder 3, wobei die Reste R² verschieden sein können, wenn m den Wert 2 oder 3 hat;
R¹
   Wasserstoff; C₁-C₅-Alkyl, C₃-C₅-Alkenyl, C₃-C₅-Alkinyl, C₁-C₄-Alkoxy, wobei diese Gruppen ein bis zwei Halogenatome tragen können;
R²
   Cyano, Halogen, C₁-C₄-Alkyl, C₁-C₄-Alkoxy, C₁-C₄-Halogenalkyl;
R³
   C₁-C₁₂-Alkyl; C₁-C₁₂-Halogenalkyl; C₃-C₈-Cycloalkyl; oder ein ggf. substituiertes ein- bis dreikerniges aromatisches System, welches neben Kohlenstoffatomen ein bis vier Stickstoffatome oder ein bis drei Heteroatome, ausgewählt aus einer Gruppe bestehend aus zwei Stickstoffatomen und einem Sauerstoff- oder Schwefelatom, enthalten kann, wobei die aromatischen Reste noch einen bis drei Substituenten tragen können, ausgewählt aus einer Gruppe bestehend aus Halogen; C₁-C₄-Alkyl; C₁-C₄-Alkoxy, indem man die Etherverbindung der Formel I, mit R³-CO gleich H, mit Carbonsäurehalogeniden oder Carbonsäureanhydriden in Gegenwart einer Säure umsetzt. Die Säuren wirken als Katalysatoren.

Es sind zahlreiche Verfahren zur Acylierung von Phenyl-alkyl-Ethern bekannt. Eine Übersicht über die gängigen Methoden wird in den folgenden Literaturstellen beschrieben.
1. G.A. OLAH, Friedel-Crafts and related reactions, Bd III, S. 48 - 51, 180 - 189, John Wiley and Sons Inc. New York. 1964
2. D.E. PEARSON, C.A. BUEHLER, Synthesis 1972, S. 533 - 542.

Diese Verfahren sind für eine Acylierung von Aryl-benzylethern der Formel II nicht geeignet, da unter den Reaktionsbedingungen bevorzugt eine Spaltung des Benzylethers erfolgt. Dies wird beschrieben in Houben-Weyl, Methoden der organischen Chemie Ed. 6/3 S. 146 Tabelle 13 und S. 151 Zeilen 24 ff. Georg Thieme Verlag Stuttgart 1965. Es ergibt sich auch aus eigenen Vergleichsexperimenten.

Die acylierten Aryl-benzylether werden nach den bekannten Verfahren nur in sehr geringer Ausbeute erhalten. Im Falle der Ausgangsstoffe der Formel II muß zusätzlich mit ausbeutevermindernden Nebenreaktionen durch die Anwesenheit der funktionellen Gruppierungen R¹-Y-CO-CX- gerechnet werden.

Der vorliegenden Erfindung lag daher die Aufgabe zugrunde, die Verbindungen I besser zugänglich zu machen.

Es wurde nun ein neues Verfahren zur Herstellung von Aryl-benzylethern der Formel I gefunden, welches überraschend hohe Ausbeuten liefert und welches dadurch gekennzeichnet ist, daß man Arylbenzylether der Formel II 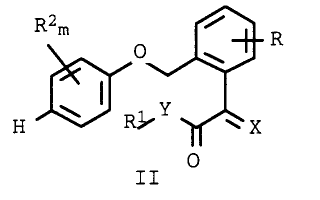 worin X, Y, R, R¹ und R² die vorstehend genannte Bedeutung haben, mit einem Carbonsäurehalogenid der Formel R³-CO-Halogen oder mit einem Carbonsäureanhydrid der Formel R³-COO-CO-R³ oder mit einem Carbonsäure-Sulfonsäureanhydrid der Formel R³-COO-SO₂-R⁴, wobei R³ die vorstehend genannte Bedeutung hat und R⁴ C₁-C₄-Alkyl, C₁-C₄-Halogenalkyl oder Aryl bedeutet, in Gegenwart einer Säure sowie ggf. in Gegenwart eines Verdünnungsmittels umsetzt.

Bei Verwendung von Carbonsäurehalogeniden als Acylierungsagentien in stöchiometrischer Menge, bezogen auf die Verbindung der Formel II oder in einem Überschuß bis zu 10 Mol-Äquivalenten über die stöchiometrische Menge hinaus werden als Katalysatoren z.B. Lewissäuren, z.B. AlCl₃, AlBr₃, ZnCl₂, ZnBr₂, SnCl₄, FeCl₃, FeBr₃, TiCl₄, GaCl₃, InCl₃, BF₃ in Mengen von 1 bis 5 Mol-Äquivalenten Säure, bezogen auf die Verbindung II, eingesetzt. Die Reaktionstemperatur wird z.B. im Bereich von -80°C bis 25°C, bevorzugt -50°C bis 0°C gewählt.

Bei Verwendung von Carbonsäureanhydriden oder Carbon-sulfonsäureanhydriden in stöchiometrischer Menge, bezogen auf die Verbindung der Formel II oder in einem Überschuß bis zu 30 Mol-Äquivalenten über die stöchiometrische Menge hinaus werden als Katalysatoren z.B. Lewis- oder Brönstedsäuren in Mengen von 1 bis 5 Mol-Äquivalenten Säure, bezogen auf die Verbindung II verwendet. Die Reaktionstemperatur wird z.B. im Bereich 0°C bis 140°C, bevorzugt 20°C bis 80°C, gewählt.

Als Verdünnungsmittel sind z.B. inerte Kohlenwasserstoffe wie Pentan, Hexan, Heptan, Octan oder chlorierte Kohlenwasserstoffe wie Dichlormethan, 1,2-Dichlorethan, Chlorbenzol, 1,2-Dichlorbenzol, 1,3-Dichlorbenzol, 1,4-Dichlorbenzol oder dipolar aprotische Lösungsmittel wie Acetonitril, Nitromethan, Nitrobenzol oder Alkylester von Alkan- und Arylcarbonsäuren wie Essigsäuremethylester, Essigsäureethylester, Essigsäurepropylester, Benzoesäuremethylester, Benzoesäureethylester geeignet.

Die als Ausgangsstoffe dienenden Arylbenzyl-Ether II sind bekannt und ihre Herstellung beschrieben (vgl. EP 178 826, EP 253 213, EP 254 426, EP 280 185, EP 336 211, EP 348 766, EP 498 188, EP 398 692 und EP 493 711).

Das erfindungsgemäße Herstellungsverfahren läßt sich mit Erfolg zur Synthese der definitionsgemäßen Verbindungen I anwenden, vor allem zur Herstellung solcher Verbindungen in denen die Variablen folgende Bedeutung haben:
R
   Wasserstoff, Halogen wie Fluor, Chlor, Brom und Iod, insbesondere Fluor und Chlor; Cyano; C₁-C₄-Alkyl wie Methyl, Ethyl, Isopropyl und n-Propyl, insbesondere Methyl und Ethyl; C₁-C₄-Alkoxy wie Methoxy, Ethoxy, 1-Methylethoxy und n-Propoxy insbesondere Methoxy und Ethoxy;
X
   CH₂, CH-CH₃, CH-CH₂CH₃, CH-OCH₃, N-OCH₃; Y
   Sauerstoff, Schwefel, direkte Bindung oder Stickstoff, wobei der Stickstoff ein Wasserstoffatom, eine C₁-C₄-Alkylgruppe wie vorstehend im allgemeinen und insbesondere genannt, eine C₁-C₄-Alkoxygruppe wie vorstehend im allgemeinen und insbesondere genannt, tragen kann;
m
   0, 1, 2 oder 3, wobei die Reste R² verschieden sein können, wenn m den Wert 2 oder 3 hat;
R¹
   Wasserstoff; C₁-C₅-Alkyl, besonders C₁-C₄-Alkyl wie vorstehend im allgemeinen und insbesondere genannt; C₃-C₅-Alkenyl wie 1-Propenyl, 2-Propenyl, 1-Methylethenyl, 1-Butenyl, 2-Butenyl, 3-Butenyl, 3-Methyl-2-butenyl, insbesondere 2-Propenyl, 2-Butenyl und 3-Methyl-2-butenyl; C₃-C₅-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, insbesondere 2-Propinyl und 2-Butinyl, C₁-C₄-Alkoxy wie Methoxy, Ethoxy, 1-Propoxy, 2-Propoxy, 2-Methyl-1-propoxy, 2-Methyl-2-propoxy, 1-Butoxy und 2 Butoxy;
   wobei diese Kohlenwasserstoff-Gruppen ein bis zwei Halogenatome wie Fluor, Chlor, Brom und Iod, insbesondere Fluor und Chlor tragen können;
R¹ bedeutet außerdem Vinyl oder Ethinyl, wenn Y eine direkte Bindung bedeutet;
R²
   Cyano; Halogen wie vorstehend im allgemeinen und insbesondere genannt; C₁-C₄-Alkyl wie vorstehend im allgemeinen und insbesondere genannt; C₁-C₄-Alkoxy wie vorstehend im allgemeinen und insbesondere genannt; C₁-C₄-Halogenalkyl besonders C₁-C₂-Halogenalkyl wie Chlormethyl, Dichlormethyl, Trichlormethyl, Fluormethyl, Difluormethyl, Trifluormethyl insbesondere Trifluormethyl;
R³
   C₁-C₁₂-Alkyl, besonders C₁-C₇-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, Heptyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl;
C₁-C₁₂-Halogenalkyl, besonders C₁-C₇-Halogenalkyl wie Chlormethyl, Brommethyl, Fluormethyl, Trichlormethyl, Trifluormethyl, Difluormethyl, 2-Chlorethyl, 2-Bromethyl, 2-Fluormethyl, 2,2,2-Trichlorethyl, 3-Chlorpropyl, 3-Brompropyl, 4-Chlorbutyl, 4-Brombutyl, 1-Chlorethyl, 1-Bromethyl, 1-Fluorethyl, 5-Chlorpentyl, 5-Brompentyl, 6-Chlorhexyl, 6-Bromhexyl, 7-Chlorheptyl und 7-Bromheptyl;
C₃-C₈-Cycloalkyl, besonders C₃-C₆-Cycloalkyl wie Cyclopropyl, Cyclobutyl, Cyclopentyl und Cyclohexyl;
Ein ggf. subst. ein- bis dreikerniges aromatisches System, welches neben Kohlenstoffatomen ein bis vier Stickstoffatome oder ein oder zwei Stickstoffatome und ein Sauerstoff- oder Schwefelatom enthalten kann, besonders Phenyl, 1-Naphthyl, 2-Naphthyl, 1-Anthryl, 2-Anthryl, 9-Anthryl, 9-Fluorenyl und 2-Fluorenyl, insbesondere Phenyl, fünfring Heteroaromaten enthaltend ein bis drei Heteroatome, ausgewählt aus einer Gruppe bestehend aus drei Stickstoffatomen und einem Sauerstoff- oder Schwefelatom, wie 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Triazol-2-yl, 1,3,4-Oxadiazol-2-yl, 1,3,4-Thiadiazol-2-yl, 1,3,4-Triazol-2-yl, 1,2,3-Oxadiazol-4-yl, 1,2,3-Oxadiazol-5-yl, 1,2,5-Triazol-3-yl, 1,2,3-Triazol-4-yl, 1,2,3-Triazol-5-yl, 1,2,3-Triazol-4-yl, 5-Tetrazolyl, 1,2,3,4-Thiatriazol-5-yl und 1,2,3,4-Oxatriazol-5-yl, insbesondere 3-Pyrrolyl, 3-Isoxazolyl, 5-Isoxazolyl, 4-Oxazolyl, 4-Thiazolyl, 4-Pyrazolyl, 5-Pyrazolyl, 1,3,4-Oxadiazol-2-yl und 1,3,4-Thiadiazol-2-yl, Sechsring Heteroaromaten enthaltend ein bis vier Stickstoffatome als Heteroatome wie 2-Pyridinyl, 3-Pyridinyl, 4-Pyridinyl, 3-Pyridazinyl, 4-Byridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl, insbesondere 2-Pyridinyl, 3-Pyridinyl, 2-Pyrimidinyl, 4-Pyrimidinyl und 1,3,5-Triazin-2-yl, wobei diese Gruppen ein bis drei Halogenatome wie Chlor, Fluor, Brom und Iod, insbesondere Chlor und Fluor, und/oder einen bis drei C₁-C₄-Alkyl-Reste wie vorstehend im allgemeinen und im besonderen genannt, und/oder einen bis drei C₁-C₄-Alkoxy-Reste wie vorstehend im allgemeinen und im besonderen genannt, tragen können.

Die Ketone der allgemeinen Formel I sind wertvolle Zwischenprodukte für die Synthese der E- und Z-Oximether der allgemeinen Formel III, die im Pflanzenschutz als Schädlingsbekämpfungsmittel vorzugsweise als Fungizide Verwendung finden (vgl. DE 3937 457, EP 513 580, EP 498 188). 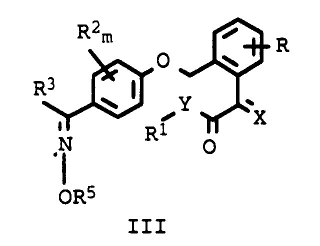 Wobei R⁵
C₁-C₆-Alkyl, C₂-C₆-Alkenyl und C₂-C₆-Alkinyl bedeutet, wobei diese Reste ein bis drei Halogenatome und/oder einen bis drei der folgenden Reste tragen können:
Cyan; Nitro; C₁-C₆-Alkoxy, C₁-C₆-Alkylthio und C₃-C₆-Cycloalkyl; R⁵ bedeutet außerdem ein 5- oder 6-gliedriges aromatisches System, vorzugsweise Phenyl, welches seinerseits einen bis drei Substituenten tragen kann, ausgewählt aus einer Gruppe bestehend aus Halogen, Cyano, Nitro, C₁-C₄-Alkyl und C₁-C₄-Alkoxy.

Die Endprodukte III lassen sich nach allgemein üblichen Verfahren und Methoden, ausgehend von den Ketonen der Formel I, herstellen (vgl. DE 39 37 457, EP 513 580, EP 498 188).

### Vergleichsexperimente

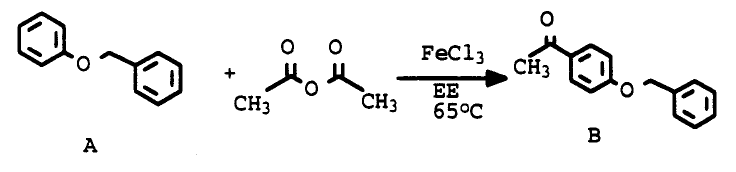

9,21 g (50 mmol) Benzylphenylether A und 10,21 g (100 mmol) Essigsäureanhydrid (Ac₂O) werden zu einer Lösung von 2,43 g (15 mmol) FeCl₃ in 50 ml Essigester bei 65°C unter Rühren zugetropft. Nach 1 Std. wird die Mischung im Gaschromatographen (GC) analysiert. Ergebnis: unselektive Reaktion, 80 % des Benzylphenylethers sind verbraucht, die Reaktionsmischung enthält weniger als 1 % der Verbindung B.

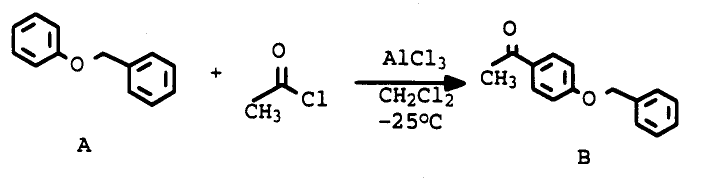

4 g (30 mmol) AlCl₃ werden in 25 ml CH₂Cl₂ bei -25°C unter Rühren vorgelegt. Man tropft bei -25°C 2,40 g (30 mmol) Acetylchlorid (AcCl) zu. Nach 15 Min. werden 5,53 g (30 mmol) Benzylphenylether A in 10 ml CH₂Cl₂ zugetropft.

Nach 1 Std. wird hydrolysiert und im GC analysiert. Ergebnis: völlig unselektive Reaktion. 83 % Benzylphenylether A sind verbraucht, die Reaktionsmischung enthält weniger als 1 % Verbindung B.

### Allgemeine Vorschrift zur erfindungsgemäßen Acylierung mit AlCl₃

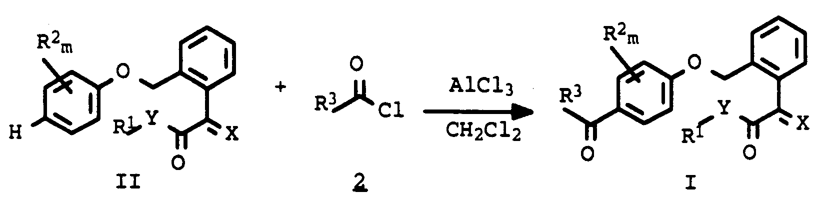

75 mmol AlCl₃ werden in 75 ml CH₂Cl₂ bei -15°C vorgelegt. Innerhalb 15 Min. werden 75 mmol Säurechlorid 2 bei -15°C zugetropft. Man kühlt auf -20 bis -25°C und tropft eine Lösung von 30 mmol Arylbenzylether II in 20 ml CH₂Cl₂ zu. Man rührt 1 Std. nach, hydrolysiert auf 200 g Eis, gibt 150 ml CH₂Cl₂ zu und trennt die Phasen. Nach Trocknung über Na₂SO₄ wird im Vakuum eingeengt und der Rückstand umkristallisiert. Man erhält die Verbindung I.

Nach der oben beschriebenen Methode wurden die folgenden Ergebnisse erhalten.

| X | R²ₘ | R³ | Y-R¹ | Lösungsmittel für Kristallisationen | Ausbeute an Verbind. I bezogen auf Verbind. II |
|---|---|---|---|---|---|
| NOCH₃ | 2-CH₃ | CH₃ | OCH₃ | Toluol/Heptan=1/1, | 85 % |
| NOCH₃ | 2-CH₃ | C₂H₅ | OCH₃ | Toluol/Heptan=1/2 | 82 % |
| NOCH₃ | 2-CH₃ | ClCH₂CH₂ | OCH₃ | CH₃OH | 58 % |
| NOCH₃ | 2-CH₃ | ClCH₂CH₂CH₂ | OCH₃ | CH₃OH | 65 % |
| NOCH₃ | 2-CH₃ | ClCH₂CH₂CH₂CH₂ | OCH₃ | CH₃OH | 70 % |
| NOCH₃ | 2-CH₃ | Ph | OCH₃ | Toluol/Heptan=1/2,5 | 81 % |
| NOCH₃ | 2,5-(CH₃)₂ | CH₃ | OCH₃ | Toluol/Heptan=1/2 | 83 % |
| NOCH₃ | 2-CH₃ | CH₃ | NHCH₃ | Toluol/Heptan=1/2 | 91 % |

### Katalytische Acylierung mit FeCl₃

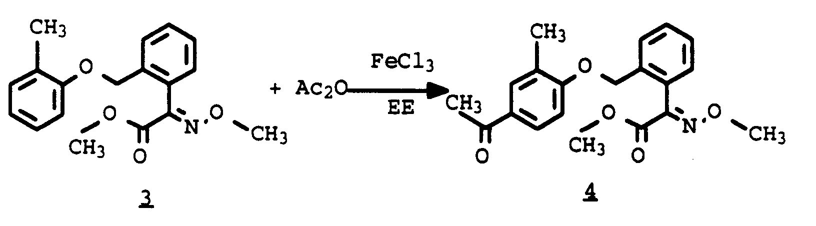

490 mg (3 mmol) FeCl₃ werden in 10 ml Essigester bei 65°C vorgelegt. Man tropft eine Mischung aus 3,13 g (10 mmol) Arylbenzylether 3 und 2,04 g (20 mmol) Ac₂O in 10 ml Essigester zu. Nach 16 Std. bei 65°C wird im Vakuum eingeengt und der Rückstand an Kieselgel chromatographiert. Man erhält 2,42 g Verbindung 4 in 68 % Ausbeute.

In entsprechender Weise werden die in den folgenden Tabellen genannten Verbindungen erhalten.

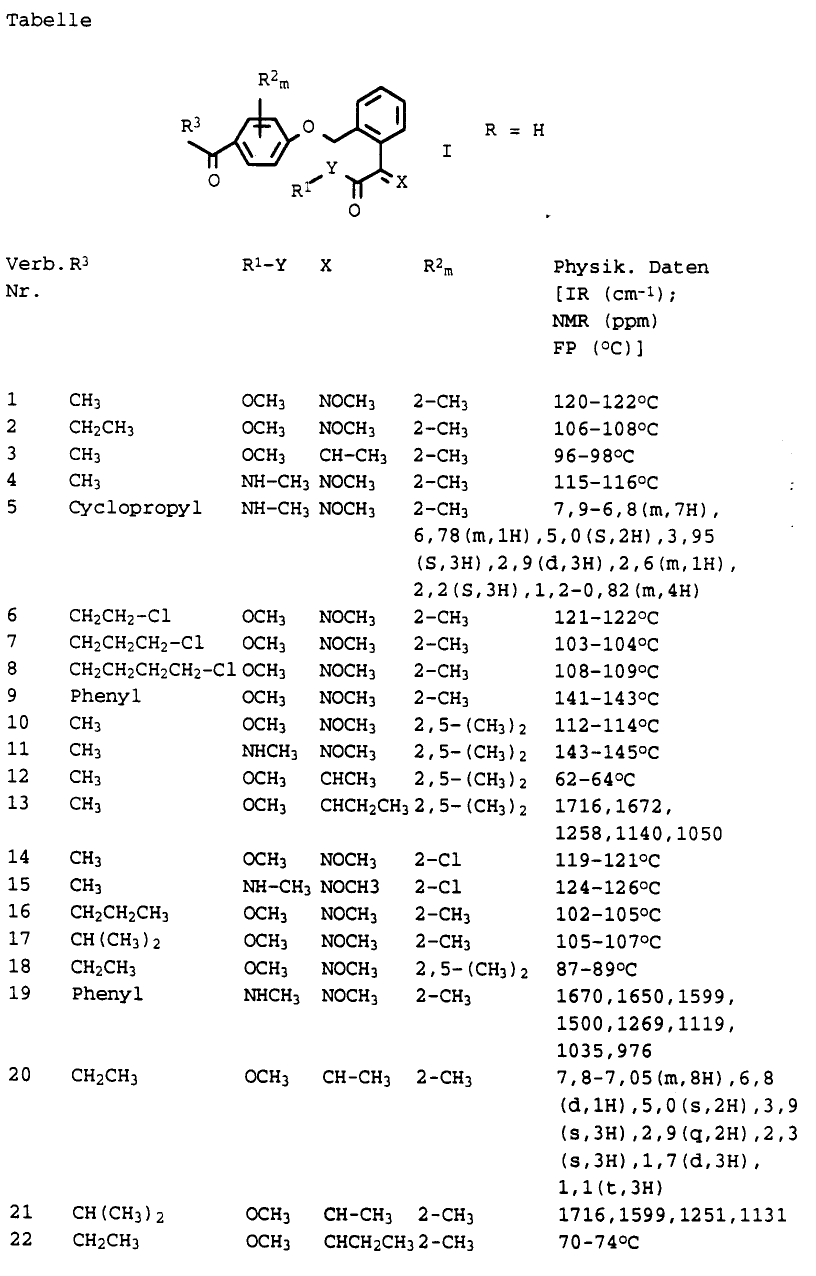

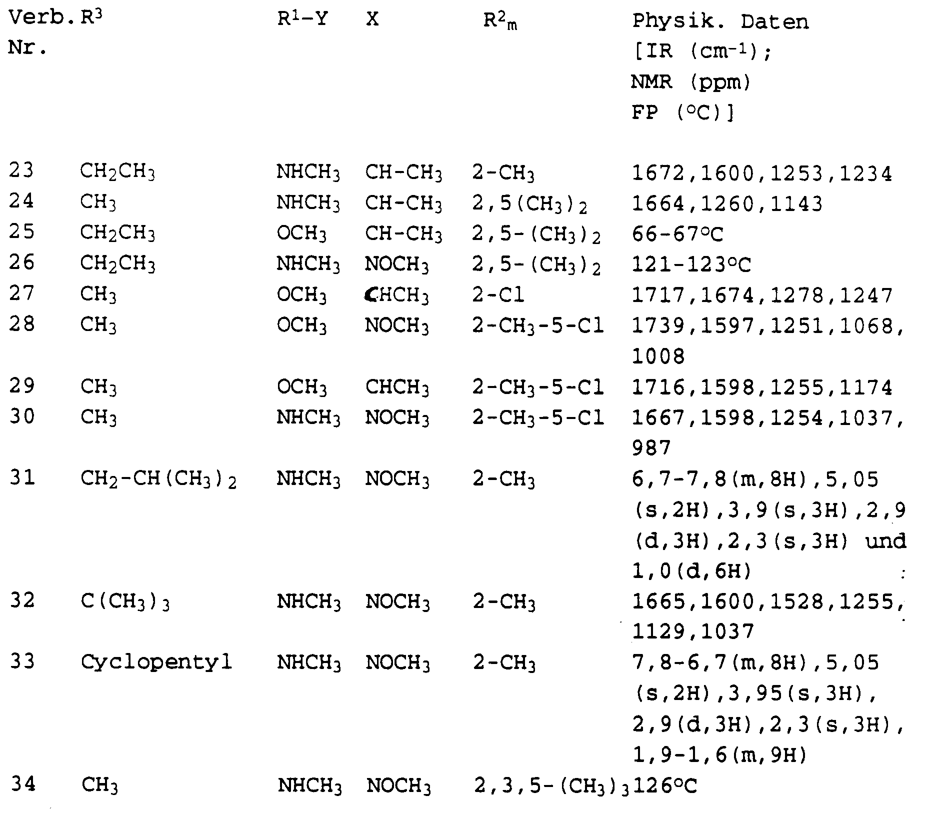

### Tabelle 1

Verbindungen der Formel I.1, in denen Y-R¹ für Methoxy steht und die Kombination der Substituenten R³ und für eine Verbindung jeweils einer Zeile einer Spalte der Tabelle A entspricht

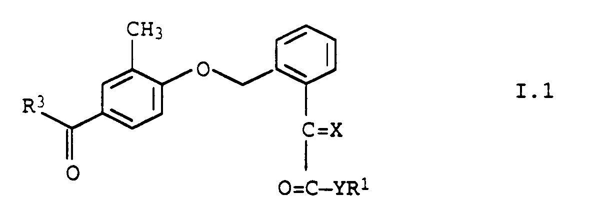

### Tabelle 2

Verbindungen der Formel I.1, in denen Y-R¹ für Methylamino steht und die Kombination der Substituenten R³ und X für eine Verbindung jeweils einer Zeile einer Spalte der Tabelle A entspricht.

### Tabelle 3

Verbindungen der Formel I.2, in denen Y-R¹ für Methoxy steht und die Kombination der Substituenten R³ und X für eine Verbindung jeweils einer Zeile einer Spalte der Tabelle A entspricht.

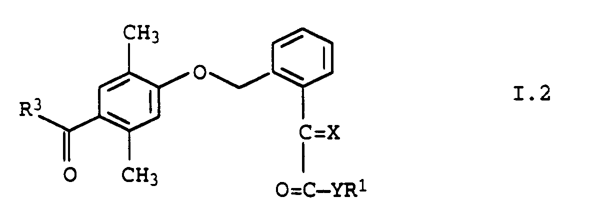

### Tabelle 4

Verbindungen der Formel I.2, in denen Y-R¹ für Methylamino steht und die Kombination der Substituenten R³ und X für eine Verbindung jeweils einer Zeile einer Spalte der Tabelle A entspricht.

### Tabelle 5

Verbindungen der Formel I.3, in denen Y-R¹ für Methoxy steht und die Kombination der Substituenten R³ und X für eine Verbindung jeweils einer Zeile einer Spalte der Tabelle A entspricht.

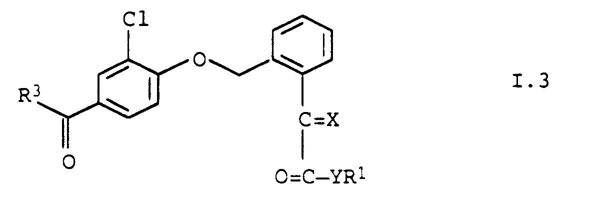

### Tabelle 6

Verbindungen der Formel I.3, in denen Y-R¹ für Methylamino steht und die Kombination der Substituenten R³ und X für eine Verbindung jeweils einer Zeile einer Spalte der Tabelle A entspricht.

### Tabelle 7

Verbindungen der Formel I.4, in denen Y-R¹ für Methylamino steht und die Kombination der Substituenten R³ und X für eine Verbindung jeweils einer Zeile einer Spalte der Tabelle A entspricht.

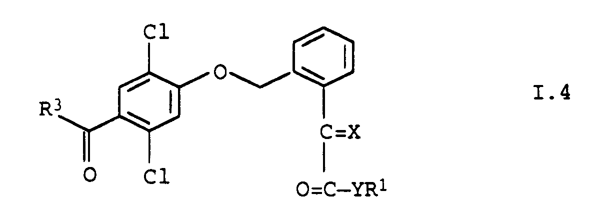

### Tabelle 8

Verbindungen der Formel I.4, in denen Y-R¹ für Methylamino steht und die Kombination der Substituenten R³ und X für eine Verbindung jeweils einer Zeile einer Spalte der Tabelle A entspricht.

### Tabelle 9

Verbindungen der Formel I.5, in denen Y-R¹ für Methylamino steht und die Kombination der Substituenten R³ und X für eine Verbindung jeweils einer Zeile einer Spalte der Tabelle A entspricht.

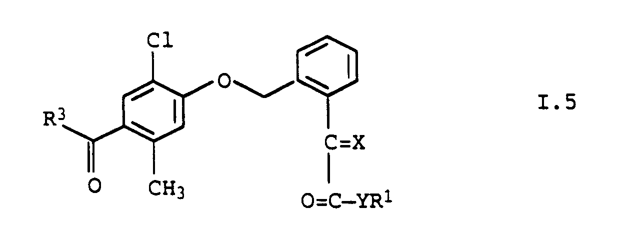

### Tabelle 10

Verbindungen der Formel I.5, in denen Y-R¹ für Methylamino steht und die Kombination der Substituenten R³ und X für eine Verbindung jeweils einer Zeile einer Spalte der Tabelle A entspricht.

### Tabelle 11

Verbindungen der Formel I.6, in denen Y-R¹ für Methylamino steht und die Kombination der Substituenten R³ und X für eine Verbindung jeweils einer Zeile einer Spalte der Tabelle A entspricht.

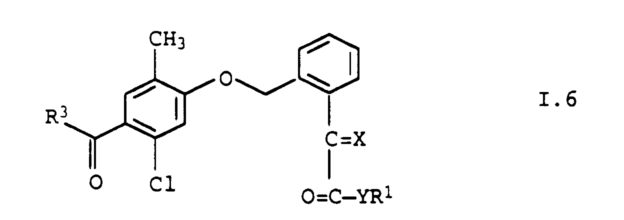

### Tabelle 12

Verbindungen der Formel I.6, in denen Y-R¹ für Methylamino steht und die Kombination der Substituenten R³ und X für eine Verbindung jeweils einer Zeile einer Spalte der Tabelle A entspricht.

### Tabelle 13

Verbindungen der Formel I.7, in denen Y-R¹ für Methylamino steht und die Kombination der Substituenten R³ und X für eine Verbindung jeweils einer Zeile einer Spalte der Tabelle A entspricht.

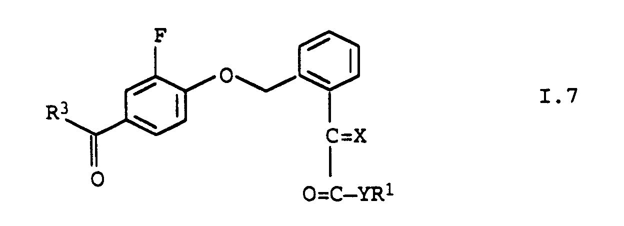

### Tabelle 14

Verbindungen der Formel I.7, in denen Y-R¹ für Methylamino steht und die Kombination der Substituenten R³ und X für eine Verbindung jeweils einer Zeile einer Spalte der Tabelle A entspricht.

### Tabelle 15

Verbindungen der Formel I.1, in denen X für NOCH₃ steht und die Kombination der Substituenten YR¹ und R³ für eine Verbindung jeweils einer Zeile einer Spalte der Tabelle B entspricht.

### Tabelle 16

Verbindungen der Formel I.2, in denen X für NOCH₃ steht und die Kombination der Substituenten YR¹ und R³ für eine Verbindung jeweils einer Zeile einer Spalte der Tabelle B entspricht.

### Tabelle 17

Verbindungen der Formel I.3, in denen X für NOCH₃ steht und die Kombination der Substituenten YR¹ und R³ für eine Verbindung jeweils einer Zeile einer Spalte der Tabelle B entspricht.

### Tabelle 18

Verbindungen der Formel I.4, in denen X für NOCH₃ steht und die Kombination der Substituenten YR¹ und R³ für eine Verbindung jeweils einer Zeile einer Spalte der Tabelle B entspricht.

### Tabelle 19

Verbindungen der Formel I.5, in denen X für NOCH₃ steht und die Kombination der Substituenten YR¹ und R³ für eine Verbindung jeweils einer Zeile einer Spalte der Tabelle B entspricht.

### Tabelle 20

Verbindungen der Formel I.6, in denen X für NOCH₃ steht und die Kombination der Substituenten YR¹ und R³ für eine Verbindung jeweils einer Zeile einer Spalte der Tabelle B entspricht.

### Tabelle 21

Verbindungen der Formel I.7, in denen X für NOCH₃ steht und die Kombination der Substituenten YR¹ und R³ für eine Verbindung jeweils einer Zeile einer Spalte der Tabelle B entspricht.

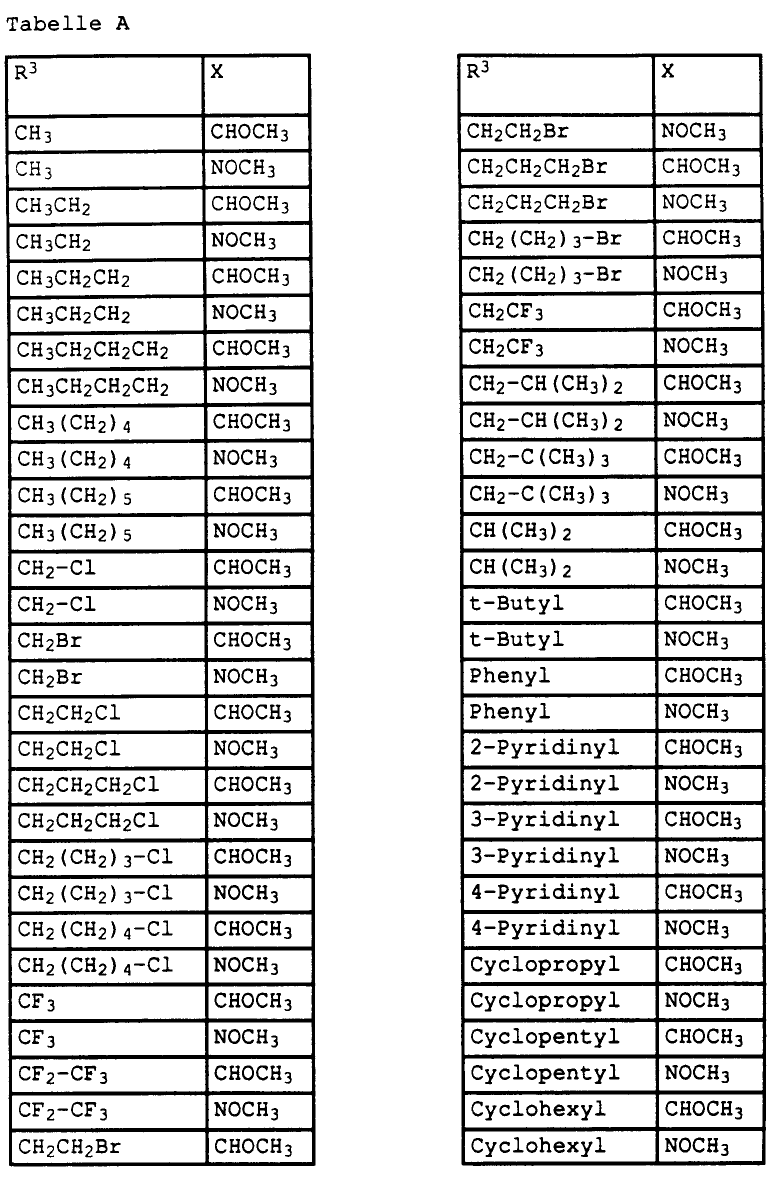

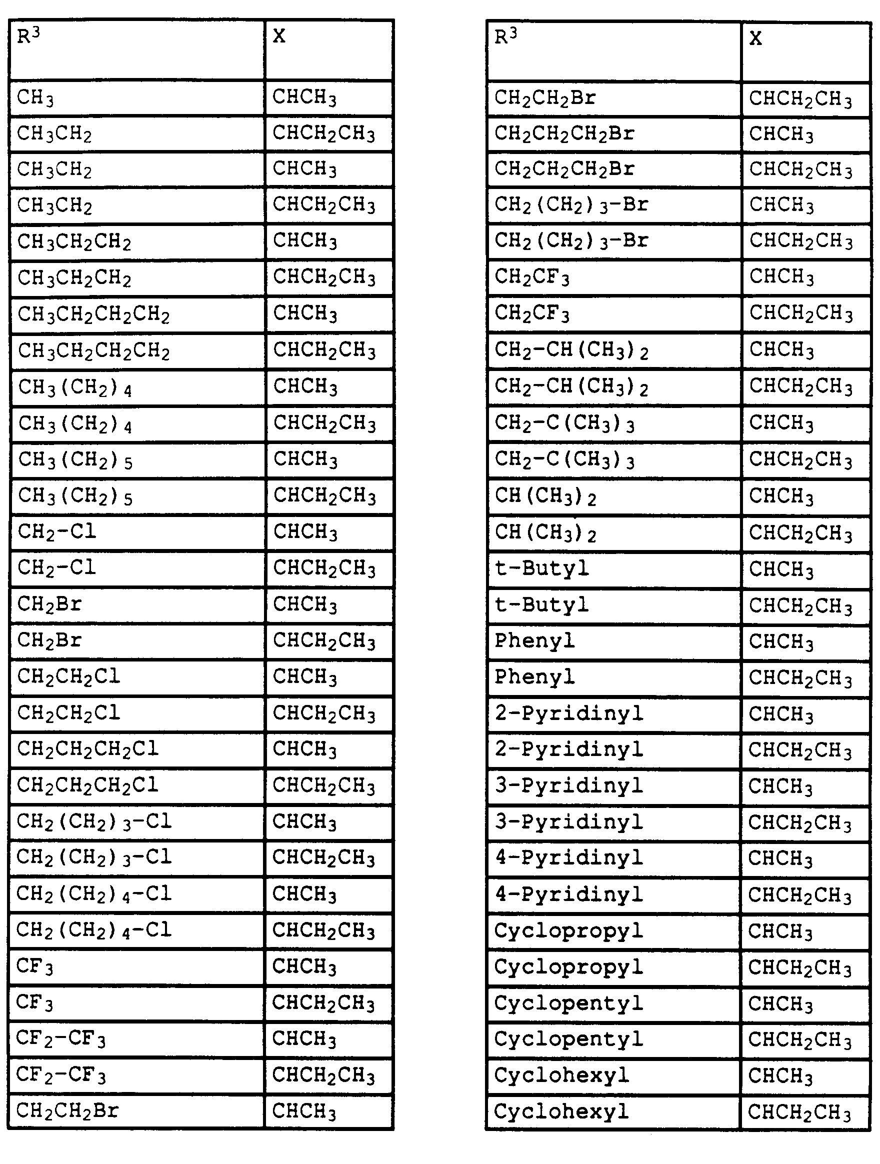

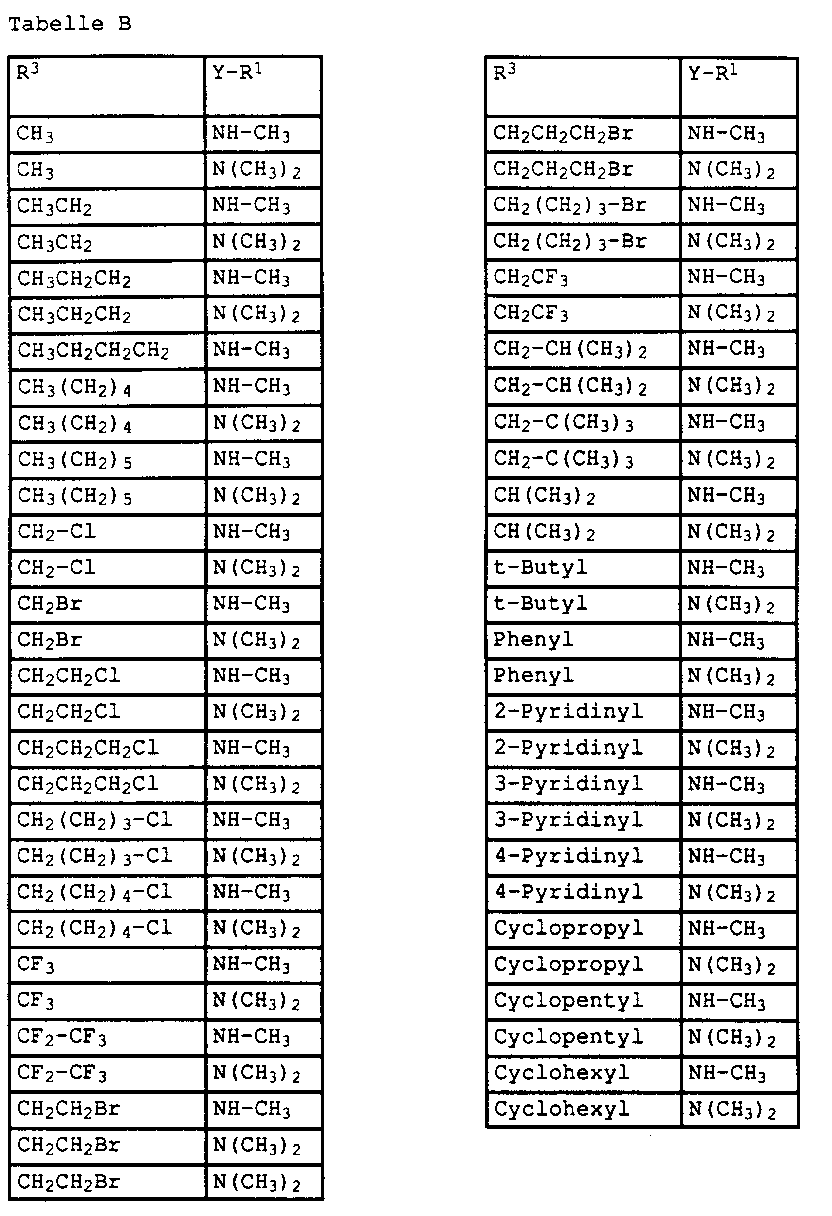

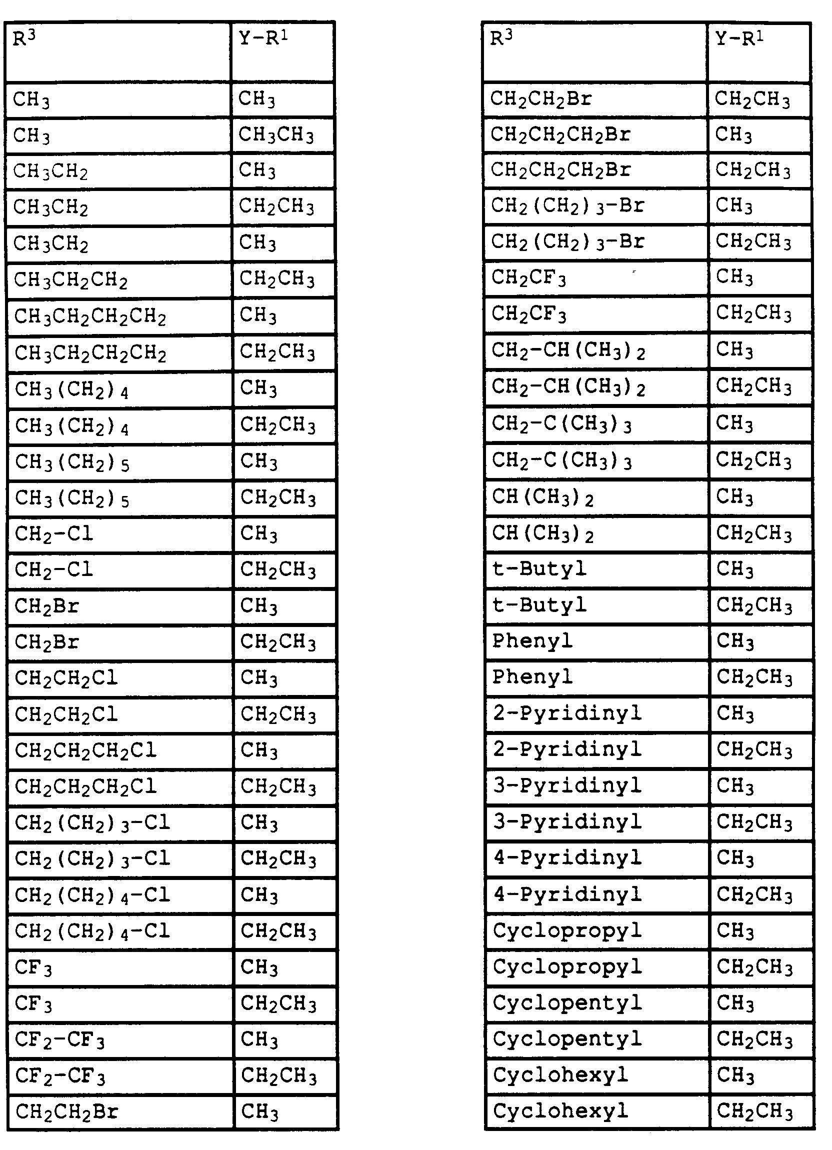

## Patentansprüche

1. Verfahren zur Herstellung von Aryl-benzylethern der allgemeinen Formel I 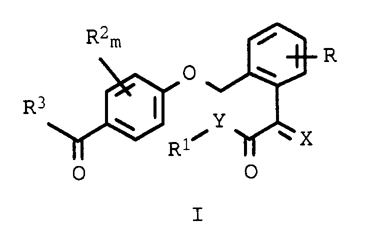 wobei die Variablen die folgende Bedeutung haben:
R
Wasserstoff, Halogen, Cyano;
C₁-C₄-Alkyl, C₁-C₄-Alkoxy;
X
CH₂, CH-CH₃, CH-CH₂-CH₃, CH-OCH₃, N-OCH₃;
Y
Sauerstoff, Schwefel, direkte Bindung oder Stickstoff, wobei der Stickstoff ein Wasserstoffatom, eine C₁-C₄-Alkylgruppe oder eine C₁-C₄-Alkoxygruppe tragen kann;
m
0, 1, 2 oder 3, wobei die Reste R² verschieden sein können, wenn m den Wert 2 oder 3 hat;
R¹
Wasserstoff; C₁-C₅-Alkyl, C₃-C₅-Alkenyl, C₃-C₅-Alkinyl, C₁-C₄-Alkoxy, wobei diese Gruppen ein bis zwei Halogenatome tragen können;
R²
Cyano, Halogen, C₁-C₄-Alkyl, C₁-C₄-Alkoxy, C₁-C₄-Halogenalkyl;
R³
C₁-C₁₂-Alkyl; C₁-C₁₂-Halogenalkyl; C₃-C₈-Cycloalkyl; oder ein ggf. substituiertes ein- bis dreikerniges aromatisches System, welches neben Kohlenstoffatomen ein bis vier Stickstoffatome oder ein bis drei Heteroatome, ausgewählt aus der Gruppe bestehend aus zwei Stickstoffatomen und einem Sauerstoff- oder Schwefelatom, enthalten kann, wobei die aromatischen Reste noch einen bis drei Substituenten tragen können, ausgewählt aus der Gruppe bestehend aus Halogen; C₁-C₄-Alkyl; C₁-C₄-Alkoxy;
dadurch gekennzeichnet, daß man einen Arylbenzyl-Ether der allgemeinen Formel II, 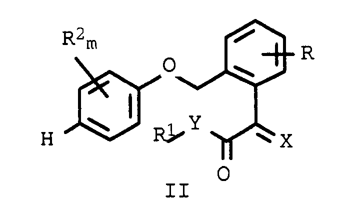 wobei X, Y, R, R¹, R² und m die oben genannte Bedeutung haben, mit einem Carbonsäurehalogenid der Formel R³-CO-Halogen oder mit einem Carbonsäureanhydrid der Formel R³-COO-CO-R³ oder mit einem Carbonsäure-Sulfonsäureanhydrid der Formel R³COO-SO₂-R⁴, wobei R³ die oben genannte Bedeutung hat und R⁴ C₁-C₄-Alkyl, C₁-C₄-Halogenalkyl oder Aryl bedeutet, in Gegenwart einer Säure sowie gegebenenfalls in Gegenwart eines Verdünnungsmittels umsetzt.

2. Verfahren nach Anspruch 1, dadurch gekennzeichnet, daß man als Säure Lewissäuren wie AlCl₃, AlBr₃, ZnCl₂, ZnBr₂, FeCl₃, FeBr₃, TiCl₄, SnCl₄, GaCl₃, InCl₃, BF₃ verwendet.

3. Verfahren nach Anspruch 1, dadurch gekennzeichnet, daß man als Säure Brönstedsäuren wie Trifluormethansulfonsäure, Methansulfonsäure, Polyphosphorsäure, Schwefelsäure verwendet.

4. Verfahren nach Anspruch 1, dadurch gekennzeichnet, daß man als Säure sulfonsaure Ionenaustauscherharze verwendet.

5. Verfahren nach Anspruch 1, dadurch gekennzeichnet, daß man als Säure saure Zeolithe oder Schichtzeolite wie z.B. Zeolith ZSM, Montmorillonit KSF, Montmorillonit K10 verwendet.

6. Verfahren nach Anspruch 2, dadurch gekennzeichnet, daß die Lewissäuren in Form der Metallverbindungen an einen inerten Träger wie Aluminiumoxid, Siliciumdioxid, Aktivkohle, Schichtsilikate oder Zeolite gebunden sind.

7. Verfahren nach einem der Ansprüche 1 bis 6, dadurch gekennzeichnet, daß man ein Carbonsäurehalogenid der Formel R³-CO-Halogen einsetzt und die Umsetzung in einem Temperaturbereich von -80°C bis +25°C durchführt.

8. Verfahren nach einem der Ansprüche 1 bis 6, dadurch gekennzeichnet, daß man ein Carbonsäureanhydrid oder ein Carbonsäure-Sulfonsäureanhydrid einsetzt und die Umsetzung bei einer Temperatur im Bereich von 0°C bis 140°C durchführt.

## Claims

1. A process for preparing aryl benzyl ethers of the general formula I 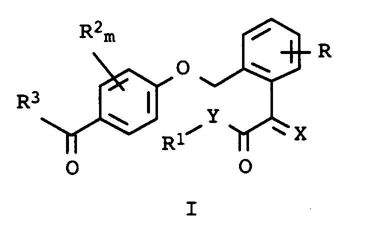 where
R is hydrogen, halogen, cyano; C₁-C₄-alkyl, C₁-C₄-alkoxy;
X is CH₂, CH-CH₃, CH-CH₂-CH₃, CH-OCH₃ or N-OCH₃;
Y is oxygen, sulfur, direct linkage or nitrogen which can carry a hydrogen atom, a C₁-C₄-alkyl group or a C₁-C₄-alkoxy group;
m is 0, 1, 2 or 3, it being possible for the R² radicals to be different when m is 2 or 3;
R¹ is hydrogen; C₁-C₅-alkyl, C₃-C₅-alkenyl, C₃-C₅-alkynyl or C₁-C₄-alkoxy, it being possible for these groups to carry one or two halogen atoms;
R² is cyano, halogen, C₁-C₄-alkyl, C₁-C₄-alkoxy or C₁-C₄-haloalkyl;
R³ is C₁-C₁₂alkyl; C₁-C₁₂-haloalkyl; C₃-C₈-cycloalkyl; or an unsubstituted or substituted mono- to trinuclear aromatic system which may, besides carbon atoms, contain one to four nitrogen atoms or one to three hetero atoms selected from the group comprising two nitrogen atoms and one oxygen or sulfur atom, it also being possible for the aromatic radicals to carry one to three substituents selected from the group comprising halogen; C₁-C₄-alkyl; C₁-C₄-alkoxy;
which comprises reacting an aryl benzyl ether of the general formula II 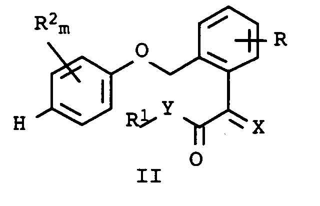 where X, Y, R, R¹, R² and m have the abovementioned meanings, with a carbonyl halide of the formula R³-CO-halogen or with a carboxylic anhydride of the formula R³-COO-CO-R³ or with a carboxylic sulfonic anhydride of the formula R³COO-SO₂-R⁴ where R³ has the abovementioned meaning, and R⁴ is C₁-C₄-alkyl, C₁-C₄-haloalkyl or aryl, in the presence of an acid and in the presence or absence of a diluent.

2. A process as claimed in claim 1, wherein Lewis acids such as AlCl₃, AlBr₃, ZnCl₂, ZnBr₂, FeCl₃, FeBr₃, TiCl₄, SnCl₄, GaCl₃, InCl₃ and BF₃ are used as acid.

3. A process as claimed in claim 1, wherein Brönsted acids such as trifluoromethanesulfonic acid, methanesulfonic acid, polyphosphoric acid and sulfuric acid are used as acid.

4. A process as claimed in claim 1, wherein ion exchange resins with sulfo groups are used as acid.

5. A process as claimed in claim 1, wherein acid zeolites or sheet zeolites such as zeolite ZSM, montmorillonite KSF and montmorillonite K10 are used as acid.

6. A process as claimed in claim 2, wherein the Lewis acids are bound in the form of the metal compounds to an inert carrier such as alumina, silica, active carbon, sheet silicates or zeolites.

7. A process as claimed in any of claims 1 to 6, wherein a carbonyl halide of the formula R³-CO-halogen is used and the reaction is carried out at from -80°C to +25°C.

8. A process as claimed in any of claims 1 to 6, wherein a carboxylic anhydride or a carboxylic sulfonic anhydride is used and the reaction is carried out at from 0°C to 140°C.

## Revendications

1. Procédé pour la préparation d'éthers aryl-benzyliques de formule générale I 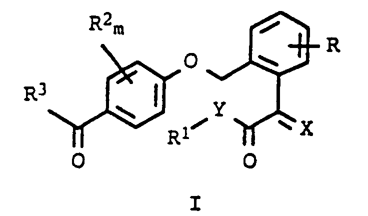 dans laquelle les symboles ont les significations suivantes :
R
l'hydrogène, un halogène, un groupe cyano ;
un groupe alkyle en C1-C4, alcoxy en C1-C4 ;
X
CH₂, CH-CH₃, CH-CH₂-CH₃, CH-OCH₃, N-OCH₃ ;
Y
l'oxygène, le soufre, une liaison directe ou l'azote, ce dernier pouvant porter un atome d'hydrogène, un groupe alkyle en C1-C4 ou un groupe alcoxy en C1-C4 ;
m
0, 1, 2 ou 3, les symboles R² pouvant avoir des significations différentes lorsque m est égal à 2 ou 3 ;
R¹
l'hydrogène ; un groupe alkyle en C1-C5, alcényle en C3-C5, alcynyle en C3-C5, alcoxy en C1-C4, ces groupes pouvant porter un à deux atomes d'halogènes ;
R²
un groupe cyano, un halogène, un groupe alkyle en C1-C4, alcoxy en C1-C4, halogénoalkyle en C1-C4 ;
R³
un groupe alkyle en C1-C12 ; halogénoalkyle en C1-C12 ; cycloalkyle en C3-C8 ; ou un système aromatique mono- à tri-cyclique éventuellement substitué qui, en plus des atomes de carbone, peut contenir un à quatre atomes d'azote ou un à trois hétéroatomes choisis dans le groupe consistant en deux atomes d'azote et un atome d'oxygène ou de soufre, les radicaux aromatiques pouvant encore porter un à trois substituants choisis parmi les halogènes, les groupes alkyle en C1-C4 et alcoxy en C1-C4,
caractérisé par le fait que l'on fait réagir un éther aryl-benzylique de formule générale II 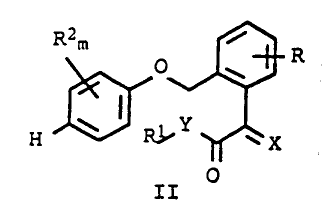 dans laquelle X, Y, R, R¹, R² et m ont les significations indiquées ci-dessus, avec un halogénure d'acide carboxylique de formule R³-CO-halogène ou avec un anhydride d'acide carboxylique de formule R³-COO-CO-R³, ou avec un anhydride mixte d'acide carboxylique et d'acide sulfonique de formule R³COO-SO₂-R⁴, R³ ayant les significations indiquées ci-dessus et R⁴ représentant un groupe alkyle en C1-C4, halogénoalkyle en C1-C4 ou aryle, en présence d'un acide et le cas échéant en présence d'un diluant.

2. Procédé selon la revendication 1, caractérisé par le fait que l'acide utilisé est un acide de Lewis tel que AlCl₃, AlBr₃, ZnCl₂, ZnBr₂, FeCl₃, FeBr₃, TiCl₄, SnCl₄, GaCl₃, InCl₃, BF₃.

3. Procédé selon la revendication 1, caractérisé par le fait que l'acide utilisé est un acide de Brönsted tel que l'acide trifluorométhanesulfonique, l'acide méthanesulfonique, l'acide polyphosphorique, l'acide sulfurique.

4. Procédé selon la revendication 1, caractérisé par le fait que l'acide utilisé consiste en une résine échangeuse d'ions acide sulfonique.

5. Procédé selon la revendication 1, caractérisé par le fait que l'acide utilisé consiste en une zéolithe acide ou une zéolithe lamellaire, par exemple la zéolite ZSM, la montmorillonite KSF, la montmorillonite K10.

6. Procédé selon la revendication 2, caractérisé par le fait que les acides de Lewis consistant en dérivés métalliques sont fixés sur un support inerte, par exemple d'alumine, de silice, de charbon actif, de silicate lamellaire ou de zéolithe.

7. Procédé selon une des revendications 1 à 6, caractérisé par le fait que l'on met en oeuvre un halogénure d'acide carboxylique de formule R³-CO-halogène et on procède à la réaction dans l'intervalle de température allant de -80 à +25°C.

8. Procédé selon une des revendications 1 à 6, caractérisé par le fait que l'on met en oeuvre un anhydride d'acide carboxylique ou un anhydride mixte d'acide carboxylique et d'acide sulfonique et on procède à la réaction dans l'intervalle de température de 0 à 140°C.
